# EUROPEAN PATENT APPLICATION

(11) **EP 4 310 856 A1**
(43) Date of publication of application: **24.01.2024**
(21) Application number: 22382689.2
(22) Date of filing: 19.07.2022
(51) Int. Cl.: G16H 40/00, G16H 10/40

(54) **PROCESSING OF TEST SAMPLES IN A LABORATORY SETTING**

(71) Applicant: F. Hoffmann-La Roche AG, 4070 Basel (CH); Roche Diagnostics GmbH, 68305 Mannheim (DE)
(72) Inventor: SUAREZ NOVAU, Luis, Barcelona, Sant Cugat del Vallès, 08174 (ES)
(74) Representative: Herren, Barbara

(57) **Abstract**

A computer-implemented method of automatically locating and processing test sample(s) 200 from a patient within a laboratory setting 100 is disclosed. The method comprises determining that a test sample 200 has not been processed by any device 300, 310, 320 in the laboratory 100 and the test sample 200 is still within a time period of processing, performing inquiry of historical data of previous non-located test samples maintained in a database 120 for possible locations of non-located test sample 200, and notifying laboratory operator of a location with a highest probability of test sample location based on the historical data.

## Description

### Technical Field

The present disclosure generally relates to locating and providing processing for delayed/missing test samples from patients within a laboratory setting within an acceptable time period for processing the patient test sample.

### Background

During normal operations of a laboratory, test samples from patients are typically received throughout the course of the day (or have been received from the day before) in a variety of different ways such as, for example, test sample collection points and satellite laboratories, via several different channels such as, for example, internal hospital communications, and with different priorities such as, for example, STAT samples. Therefore, the laboratory needs to be able to locate and identify these test samples at some point during the day as well as to manage the test samples so that there is adequate traceability and chain of custody for those test samples.

Surprisingly, sometimes the test samples from patients are not identified in the laboratory IT system as arriving at the laboratory for processing, either the patient test samples never physically arrive at the laboratory or the patient test samples were never registered in the laboratory IT system upon arriving at the laboratory. This situation can be very problematic as it has the potential of putting the patient's life in danger due to the absence of test results within the required acceptable time period. Additionally, the situation might cause serious reputational issues for the laboratory itself.

Document US Pub. 2009/0263281 discloses an automatic analyzer capable of recording contents of a sample subjected to a special treatment for an unusual error condition such as, for example wrong reagent used or sample quality issues, to ensure traceability of the sample as well as to cope with each unusual error condition. However, this system focuses on errors or problems during operation, not trying to complete the processing workflow within a desired turnaround time.

Document US Pat. 8,418,001 discloses a system and method to provide system monitoring and detailed troubleshooting workflow guidance. This system simply relates to generic troubleshooting and guidance,

Document US Pub. 2014/0282181 discloses systems and methods to visualize and manage medical device operations on an interface and to provide troubleshooting for those medical device operations. This system is simply concerned with providing information visualization.

### Summary

It is an object of the present disclosure to locate and provide processing for delayed/missing test samples from patients within a laboratory setting within an acceptable time period.

According to a first aspect of the present disclosure, a computer-implemented method of automatically locating and processing test samples from a patient within a laboratory setting is presented. The method comprises determining by a control unit that a test sample has not been identified and/or processed by any laboratory device in the laboratory and that the test sample is still within an acceptable time period of processing, performing inquiry of historical data of previous non-located and subsequently found test samples maintained in a database communicatively connected to the control unit for possible locations of non-processed test sample, and notifying laboratory operator of a location with a highest probability of non-processed test sample location based on the historical data inquiry.

The computer-implemented method further comprises searching for additional test samples from the patient within the laboratory and, if additional test samples from the patient are located within the acceptable time period for processing, processing required tests on the additional test samples.

The computer-implemented method further comprises, if the test sample is not located within the laboratory and within the acceptable time period for processing and no additional test samples from the patient are available, alerting a collection laboratory that new test samples from the patient need to be collected.

The computer-implemented method further comprises, if the test sample is not located within the laboratory and within the acceptable time period for processing, alerting a collection laboratory that new test samples from the patient need to be collected.

The computer-implemented method further comprises, if the test sample is located within the laboratory and within the acceptable time period for processing in a laboratory device connected to the laboratory transportation system, recalculating the processing steps for the test sample to determine if and what steps or results were missed and performing the recalculated processing steps on the test sample.

The computer-implemented method further comprises, if the test sample is located within the laboratory and within the acceptable time period for processing in a laboratory device not connected to the laboratory transportation system, alerting a laboratory operator to manually move the test sample to next processing step within the laboratory.

The computer-implemented method further comprises, if the test sample is located within the laboratory and within the acceptable time period for processing but a significant number of required tests cannot be performed on the test sample within the acceptable time period, alerting the laboratory operator that troubleshooting is needed for that test sample and/or that some laboratory devices need to be masked in order to perform all of the required tests within the acceptable time period.

The computer-implemented method further comprises, if the test sample is located within the laboratory and within the acceptable time period processing but a significant number of required tests are not available in the laboratory, alerting the laboratory operator that troubleshooting is needed for that test sample or that the test sample needs to be transported to another laboratory.

The non-availability of the required tests in the laboratory comprises, for example, lack of needed reagent for the required tests, lack of adequate calibration for at least one of the laboratory devices, lack of available quality control for at least one of the laboratory devices, or combinations thereof.

If the test sample is located within the laboratory and within the acceptable time period for processing, the historical data of the database is updated with results from the location of the located test sample.

According to a second aspect of the present disclosure, a computer-implemented method of automatically processing patient test samples in a laboratory setting is presented. The method comprises determining by a control unit that a test sample located in a post-analytical device with pending tests will expire before a defined period of time and retrieving the test sample from the post-analytical device in order to perform the pending tests.

The post-analytical device is an archive such as, for example, a refrigerator.

In one embodiment, the test sample is automatically retrieved from the post-analytical device. In another embodiment, the test sample is manually retrieved from the post-analytical device by a laboratory operator after the control unit notifies the laboratory operator of the test sample location.

The computer-implemented method further comprises converting the test sample into a STAT test sample after retrieval from the post-analytical device in order to accelerate the processing of the test sample before the test sample expires.

According to a third aspect of the present disclosure, a laboratory system for automatically processing patient test samples in a laboratory setting is presented. The laboratory system can comprise a plurality of laboratory devices such as, for example, pre-analytic device(s), analytic device(s), and/or post-analytic device(s). The laboratory system can also comprise a control unit that is communicatively connected to the plurality of laboratory devices. The control unit is configured to determine the workflow processing of the patient test samples by the plurality of laboratory devices once the patient test samples arrive at the laboratory from an extraction point.

The pre-analytic device(s), analytic device(s), and post-analytic device(s) can be connected to each other by a transportation system in order to automatically transport test sample(s) between the different laboratory devices.

A buffer may also be connected to the transportation system to provide additional storage for the test sample(s) during times of peak laboratory use.

In addition, other laboratory device(s) other than the plurality of laboratory devices may reside within the laboratory system that may not be connected to the transportation system.

The control unit can be communicatively connected to a database. The database can store previous historical data and patterns of previous missing patient test samples.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a block diagram of a laboratory system according to an embodiment of the present disclosure.
Fig. 2 illustrates a flow chart of a method for locating and providing processing for delayed patient test samples in a laboratory setting according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

The use of the 'a' or 'an' can be employed to describe elements and components of the embodiments herein. This is done merely for convenience and to give a general sense of the inventive concepts. This description should be read to include one or at least one and the singular includes the plural unless it is obvious that it is meant otherwise.

The term 'laboratory instrument' or "laboratory device" as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis and the like. The term 'instrument' or 'device' can cover pre-analytical instruments/devices, post-analytical instruments/devices, analytical instruments/devices and laboratory middleware.

The expression 'laboratory instrument' or 'laboratory device' as used herein can encompass any monolithic or multi-modular laboratory device comprising one or more lab-devices or operative units which can be operable to execute an analytical test on one or more biological samples.

The term 'pre-analytical instrument' or 'pre-analytical device' as used herein can comprise one or more laboratory devices for executing one or more pre-analytical processing steps on one or more biological samples, thereby preparing the samples for one or more succeeding analytical tests. A pre-analytical processing step can be, for example, a centrifugation step, a capping-, decapping- or recapping step, a sealing, de-sealing step, an aliquotation step, dilution of a sample and the like.

The term 'post-analytical instrument' or 'post-analytical device' as used herein can encompass any laboratory instrument being operable to automatically process and/or store one or more biological samples. Post-analytical processing steps may comprise a recapping step, a step for unloading a sample from an analytical system or a step for storing said sample to in a storage unit or to a unit for collecting biological waste.

The term 'analyzer' / 'analytical device' as used herein can encompass any apparatus or apparatus component configured to obtain a measurement value. An analyzer can be operable to determine via various chemical, biological, physical, optical, electro-chemical or other technical procedures a parameter value of the sample or a component thereof. An analyzer may be operable to measure the parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical instrument may comprise units/components assisting with the pipetting, dosing, and mixing of samples and/or reagents. The analyzer may comprise a reagent holding unit for holding reagents to perform the assays. Reagents may be arranged, for example, in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, tissue analyzers (including morphological stainers and histochemical stainers) used to detect the result of chemical or biological reactions or to monitor the progress of chemical or biological reactions.

The term "sample" or "test sample" as used herein can be a broad term and can be given its ordinary and customary meaning to a person of ordinary skill in the art and may not be limited to a special or customized meaning. The term specifically may refer, without limitation, to an aliquot of a substance such as a chemical or biological compound. Specifically, the sample may be or may comprise at least one biological specimen, such as one or more of: blood; blood serum; blood plasma; urine; saliva. Additionally, or alternatively, the test sample may be or may comprise a chemical substance or compound and/or a reagent. The sample may specifically be a liquid sample, such as an aliquot of a fluid substance of the chemical or biological compound. For example, the liquid sample may be or may comprise at least one pure liquid, such as a liquid substance and/or a solution containing one or more liquid substances, comprising the at least one chemical and/or the biological substance. As another example, the liquid sample may be or may comprise a liquid mixture, such as a suspension, an emulsion and/or a dispersion of one or more chemical and/or biological substances. However, other, in particular non-liquid samples can be possible. For example, the container may be a reagent container. Other sample types may be, for example, tissue, homogenized material, calibration or monitoring container-like devices may be the handling subject.

The term 'laboratory middleware' as used in the present description can refer to any physical or virtual processing device configurable to control a laboratory instrument/device or system comprising one or more laboratory instruments/devices in a way that workflow(s) and workflow step(s) can be conducted by the laboratory instrument/system. The laboratory middleware may, for example, instruct the laboratory instrument/system to conduct pre-analytical, post analytical and analytical workflow(s)/ workflow step(s). The laboratory middleware may receive information from a data management unit regarding which steps need to be performed with a certain test sample. In some embodiments, the laboratory middleware can be integral with a data management unit, can be comprised by a server computer and/or be part of one laboratory instrument/device or even distributed across multiple instruments/devices of the laboratory automation system. The laboratory middleware may, for instance, be embodied as a programmable logic controller running a computer-readable program provided with instructions to perform operations.

The term "control unit " or "workflow control unit", as used herein can be a broad term and can be given its ordinary and customary meaning to a person of ordinary skill in the art and may not be limited to a special or customized meaning. The term specifically may refer, without limitation, to an electronic device configured, specifically, by hardware and/or by software programming, for controlling the functionality of the sample processing system within the laboratory middleware. The workflow control unit may further be configured for data exchange with the at least one monitoring system and/or at least one cloud server. Specifically, the workflow control unit may be or may comprise a computing device within the laboratory middleware, such as at least one processor, configured for receiving an electronic signal, such as the at least one item of information, from the at least one monitoring system and/or the at least one cloud server, and for further evaluating the received signal. Further, the workflow control unit may be configured for controlling the functionality based on the received and evaluated signal, for example, based on the at least one item of information.

A 'data storage unit' or 'database' can be a computing unit for storing and managing data such as a memory, hard disk or cloud storage. This may involve data relating to biological/medical test sample(s) to be processed by the automated system. The data management unit may be connected to an LIS (laboratory information system) and/or an HIS (hospital information system). The data management unit can be a unit within or co-located with a laboratory instrument/device. It may be part of the laboratory middleware. Alternatively, the database may be a unit remotely located. For instance, it may be embodied in a computer connected via a communication network.

The term "transportation system" as used herein can be a broad term and can be given its ordinary and customary meaning to a person of ordinary skill in the art and may not be limited to a special or customized meaning. The term specifically may refer, without limitation, to an arbitrary system, which can be configured for moving and/or transporting and/or transferring and/or carrying objects from one position to another. Specifically, the transportation system may be configured for moving the plurality of test sample tube carriers through the test sample transportation system such as from a pre-analytical device such as, for example, a laboratory loading device, to another laboratory device of the test sample transportation system. The other laboratory device may be another pre-analytical device, an analytic device, or a post-analytical device. As an example, the transportation system may comprise at least one transport element selected from the group comprising of: a conveyor, such as a belt conveyor or a chain conveyor, or a vehicle system, such as an electronic vehicle system. The transportation system may be or may comprise a multilane transportation system having a plurality of transport elements. The transportation system may be or may comprise a plurality of parallel transport elements. The transport devices may be arranged in a common plane and/or in different planes such as on top of each other.

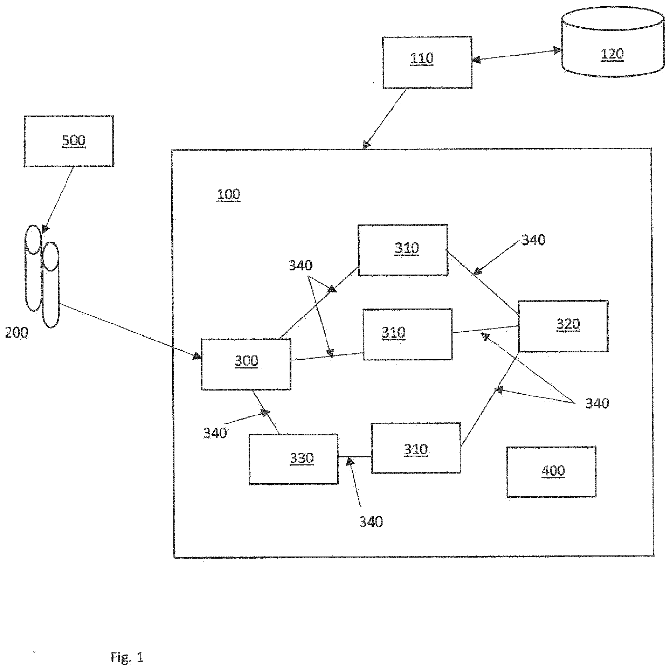

Referring initially to Fig. 1, Fig. 1 schematically illustrates a laboratory system 100. The laboratory system 100 can comprise pre-analytic device(s) 300, analytic device(s) 310, and/or post-analytic device(s) 320. The pre-analytic device(s), analytic device(s), and post-analytic device(s) can be connected to each other by a transportation system 340 in order to automatically transport test sample(s) 200 between the different laboratory devices 300, 310, 310. A buffer 330 may also be connected to the transportation system 340 to provide additional storage for the test sample(s) during times of peak laboratory use. In addition, other laboratory device(s) 400 may reside within the laboratory system 100 that may not be connected to the transportation system 340.

The laboratory system can be controlled by a control unit 110 in conjugation with the laboratory middleware. The control unit 110 determines the workflow processing of test samples 200 within the laboratory system 100. The control unit 110 can be communicatively connected to a database 120.

Test sample(s) 200 can be collected from a patient at an extraction point 500 located outside of the laboratory system 100. The extraction point 500 can notify the laboratory system 100 that the test sample(s) 200 have been collected and are en route to the laboratory system 100. The test sample(s) 200 can then be transported to the laboratory system 100 for processing by the laboratory devices 300, 310, 320. The extraction point 500 can be, for example, a satellite laboratory, a doctor's office, or any other place where a test sample(s) 200 may be extracted from a patient.

As test samples 200 arrive at the laboratory 100 for processing, the test samples 200 are identified by, for example, scanning an identification label attached to the test sample 200 by a bar code scanner and entered into the laboratory middleware system. Once the test samples 200 are entered into the laboratory middleware system, the test samples 200 are known/located within the laboratory system 100. Subsequently, if a test sample 200 is expected at a certain location within the laboratory 100 according to a workflow assigned to the test sample 200 by the laboratory middleware and is not there, the test sample 200 is deemed to be delayed or missing.

In one embodiment, a test sample 200 has been determined to be delayed but is still within an acceptable time period for processing, i.e., still within the time period where the test sample 200 can still be used for testing/processing without any test sample degradation. If the test sample 200 has not been seen/scanned at any laboratory device such as, for example, a pre-analytic device 300, an analytic device 310, or a post-analytic device 320, anywhere in the laboratory 100 during a specific time period, the presumption would be that there was problem in the collection and/or transportation of the test sample 200 from the extraction point 500 to the laboratory 100.

If it is determined that the test sample 200 never arrived at the laboratory 100 or was never entered into the laboratory middleware, the control unit 110 will first try and identify all the tests that could be done with additional test sample in the laboratory 100 from the same patient if any such test samples have reached and/or are available in the laboratory 100. If some test results can be obtained from the identified additional patient test samples, the additional test samples are retrieved and the processing workflow will proceed with the identified additional patient test samples. If possible, the additional test samples should be processed by the laboratory 100 automatically.

If no additional test samples 200 from the same patient can be located and before declaring that these test samples 200 were missing or "not received" by the laboratory 100, the laboratory system 100 can check possible locations within the laboratory 100 in which previously missing test samples 200 had been located such as, for example:
- typical places in the laboratory system 100 where incoming test samples 200 have been lost in the past based on the previous historical knowledge stored in the database 120 connected to the control unit 110. This previous historical knowledge can work as a training set in order to provide possible locations, i.e., what are the locations patterns of lost test samples in the past. This previous historical knowledge can be of particular importance for large laboratories with multiple entry points for test samples, or
- last minute received test samples 200 such as, for example, STAT samples, in a reception point of the test samples 200 in the laboratory.

An inquiry can then be formed as to where the missing test sample 200 may be located using historical knowledge from other previous episodes of missing/lost test samples 200 and where the missing/lost test samples 200 had been located. This previous historical knowledge can be inserted and stored in the database 120 communicatively connected to the control unit 110. This current scenario for the missing test sample 200 can be compared to the stored previous scenarios of missing test samples. From this comparison, it can be determined which stored scenario(s) has the highest probability of being the same or similar to the current scenario. In one embodiment, the scenario with the highest probability can then be identified by the control unit 110 and communicated to the laboratory operator by, for example, a message such as, for example, by an email, a text, an alert, and/or an alarm on a laboratory device 300, 310, 320 and/or on a personal device of the laboratory operator such as, for example, a smartphone, tablet, and/or laptop computer. In another embodiment, a list of all possible scenarios ranked in order of probability can be communicated to the laboratory operator by, for example, a message such as, for example, by an email, a text, an alert, and/or an alarm on a laboratory device 300, 310, 320 and/or on a personal device of the laboratory operator such as, for example, a smartphone, tablet, and/or laptop computer.

If none of the scenarios presented to the laboratory operator result in the missing test sample 200 being found within the laboratory and within the acceptable time period for processing the test sample 200, an alert/message can be generated by the control unit 100 and sent to the extraction point 500 notifying the extraction point 500 of the missing test sample 200 and requesting that another test sample 200 be acquired from the patient.

However, if the missing test sample 200 is found within laboratory 100 and within the acceptable time period for processing the test sample 200 in a laboratory instrument 300, 310, 340 connected to the transportation system 340, a buffer 330 connected to the transportation system 340, or the transportation system 340 itself, the processing workflow for this test sample 200 can be recalculated with emphasis on completing the previously missed processing steps in order to get the required missing test results within the acceptable time period. Then, the test sample 200 can be automatically processed according to the recalculated processing workflow to get the required pending test results or to complete the required missing processing steps such as, for example, returning the test sample 200 to a post-analytic device 320 such as, for example, an archive.

If the missing test sample 200 is found within the laboratory 100 and within the acceptable time period for processing the test sample in a non-connected laboratory device, i.e., a laboratory device 400 not connected to the transportation system 340, an alert/message can be sent to a laboratory operator to retrieve the test sample 200 from the non-connected laboratory device 400 and to manually move the test sample 200 to the laboratory device/location of the next workflow processing step. The alert/message sent to the laboratory operator can be sent by, for example, a message such as, for example, by an email, a text, an alert, and/or an alarm on a laboratory device 300, 310, 320 and/or on a personal device of the laboratory operator such as, for example, a smartphone, tablet, and/or laptop computer.

If the missing test sample 200 is found within the laboratory 100 and within the acceptable time period for processing the test sample but the specific test(s) required for the test sample 200 cannot be completed within the acceptable time period is a significant percentage of the test sample 200, i.e., greater than 50% of the required test(s), an alert/message can be sent to the laboratory operator stating that troubleshooting is needed for that test sample 200 and/or that some laboratory devices 300, 310, 320 may need to be masked in order to perform all of the required test(s) on the test sample 200 within the acceptable time period. The alert/message sent to the laboratory operator can be sent by, for example, a message such as, for example, by an email, a text, an alert, and/or an alarm on a laboratory device 300, 310, 320 and/or on a personal device of the laboratory operator such as, for example, a smartphone, tablet, and/or laptop computer.

If the missing test sample 200 is found within the laboratory 100 and within the acceptable time period for processing the test sample 200 but specific test(s) required for the test sample 200 are not currently available in the laboratory 100, an alert/message can be sent to a laboratory operator either to rectify the problem with these required test(s) or to transport the test sample 200 to another laboratory in which the specific test is available. The alert/message sent to the laboratory operator can be sent by, for example, a message such as, for example, by an email, a text, an alert, and/or an alarm on a laboratory device 300, 310, 320 and/or on a personal device of the laboratory operator such as, for example, a smartphone, tablet, and/or laptop computer.

Common reasons for the unavailability of required specific tests can be, for example, lack of needed reagent for the required specific tests on at least one of the laboratory devices 300, 310, 320, lack of adequate calibration for at least one of the laboratory devices 300, 310, 320, lack of available quality control for at least one of the laboratory devices 300, 310, 320, or combinations thereof. These common reasons can, typically, be resolved by a laboratory operator.

In another embodiment, the test sample 200 needed for the requested tests may not necessarily be delayed/missing but instead the acceptable time period for processing the test sample is about to expire. In this embodiment, the test sample 200 with pending required test results is located in the a post-analytic device 320 such as, for example an archive, i.e., a refrigerator, but the test sample 200 will expire before the defined TAT for the test sample 200 occurs within the acceptable time period for processing. In one embodiment of this scenario, the test sample 200 can be automatically retrieved from the post-analytic device 320. In another embodiment, an alert/message can be sent to the laboratory operator to manually retrieve the test sample 200 from the post-analytic device 320. In still another embodiment, the test sample 200 can be converted to a STAT sample and processed automatically/manually by the laboratory 100 as such in order to receive test results before the acceptable time period for processing the test sample 200 expires.

Another possible solution to the problem of missing test samples 200 can be to monitor the test samples 200 continuously, i.e., not only at specific time points in the laboratory 100 as is typically done, such as, for example, by cameras and sensors, in order to check for delays with respect to defined turn-around-times (TAT) and to determine the root cause of any such delay. For example, a normal test sample 200 received in the laboratory 100 could be delayed if there were some delayed intermediate steps in its processing workflow. In this example, the root cause analysis for the delay would determine which processing workflow steps caused the delay and would provide possible solutions for accelerating the workflow processing steps or replacing the current processing workflow with another alternative processing workflow. Further, in this example, in the case where the test sample 200 is delayed, it can easily be determined if the test sample 200 never reached the laboratory 100 and, therefore, is really a missing test sample 200. This example can also help in the creation of a database 120 of previous historical knowledge of the problems and remedies for use in later analysis of trends and optimization within the laboratory 100.

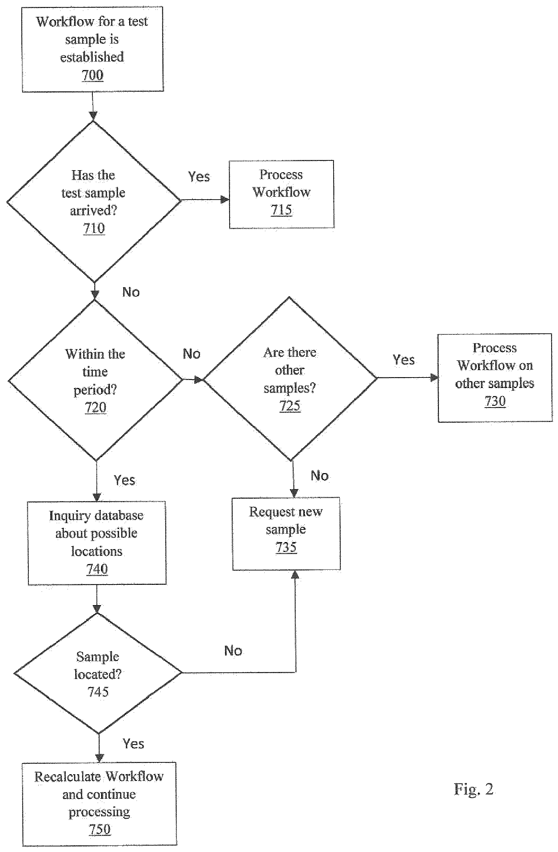

Turning to Fig. 2, Fig. 2 illustrates a flow chart for the computer-implemented method for locating and providing processing for delayed patient test samples in a laboratory setting. In step 700, a processing workflow for an incoming test sample 200 is developed in the control unit 110 of the laboratory middleware. After the processing workflow has been developed, it is determined whether the test sample 200 has arrived at the laboratory 100 in step 710. If the test sample 200 has arrived/been located in the laboratory 100, begin processing the test sample 200 according to the processing workflow in step 715.

If the test sample 200 has not arrived or cannot be located in the laboratory 100, determine, in step 720, whether the test sample 200 is still within an acceptable time period for processing the test sample 200. If it is not within the acceptable time period, determine, in step 725, whether there are other test samples 200 from the same patient available within the laboratory 100. If there are available test samples 200, proceed with the processing workflow, in step 730, with the available other test samples 200. If there are no other test samples 200 from the same patient available, request that new test samples be extracted from the patient at the extraction point 500, in step 735.

If it is still within an acceptable time period for processing the test sample 200, perform an inquiry as to possible locations for the missing test sample 200 based on previous historical data from other episodes of missing test samples in step 740.

In step 745, determine if the missing test sample 200 has been located using the prvious historical data. If the test sample 200 was not located, request that new test samples be extracted from the patient at the extraction point 500, in step 735.

If the test sample 200 was located, recalculate the processing workflow, in step 750, to ensure that all processing steps occur and proceed with processing the test sample 200 using the recalculated processing workflow.

One main advantage of this computer-implemented method is that the laboratory system can proactively provide actions to force the processing of delayed/missing test samples 200 before the expiration of the test sample 200 either in a completely automatic way, if possible, or by raising an alert for specific laboratory operators to rectify the situation. In contrast, in today's typical laboratory systems, TAT times for laboratory test samples are displayed and may indicate which test samples have been delayed. However, in these typical laboratory systems, the process to rectify the delay has to be done manually by the laboratory operator and this process must be triggered by a laboratory operator.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a paper format, or on a computer-readable data carrier on premise or located at a remote location. Specifically, the computer program product may be distributed over a data network (such as a cloud environment). Furthermore, not only the computer program product, but also the execution hardware may be located on premise or in a cloud environment.

Further disclosed and proposed is a computer-readable medium comprising instructions which, when executed by a computer system, cause a laboratory automation system to perform the method according to one or more of the embodiments disclosed herein.

Further disclosed and proposed is a modulated data signal comprising instructions, which, when executed by a computer system, cause a laboratory automation system to perform the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A computer-implemented method of automatically locating and processing test samples 200 from a patient within a laboratory setting 100, the method comprising:
determining by a control unit 110 that a test sample 200 has not been processed and/or identified by any laboratory device 300, 310, 320 in the laboratory 100 and that the test sample 200 is still within an acceptable time period of processing;
performing an inquiry by the control unit 110 of historical data of previous non-located and subsequently found test samples maintained in a database 120 communicatively connected to the control unit for possible locations of non-located test sample 200; and
notifying a laboratory operator by the control unit 110 of a location with a highest probability of non-processed test sample location based on the historical data inquiry.

2. The computer-implemented method according to claim 1, further comprising,
searching for additional test samples from the patient within the laboratory; and
if additional test samples from the patient are located within the laboratory and within an acceptable time period for processing, processing required tests on the additional test samples.

3. The computer-implemented method according to claims 1 and 2, further comprising,
if the test sample 200 is not located within the laboratory and within the acceptable time period of processing and no additional test samples for the patient are available, alerting, by the control unit 110, an extraction point 500 that new test samples from the patient need to be collected.

4. The computer-implemented method according to claims 1 and 2, further comprising,
if the test sample 200 is not located within the laboratory and within the acceptable time period of processing, alerting, by the control unit 110, an extraction point 500 that new test samples 200 for the patient need to be collected.

5. The computer-implemented method according to any of the proceeding claims, further comprising,
if the test sample 200 is located within the laboratory and within the acceptable time period for processing in a laboratory device 300, 310, 320 connected to a transportation system 340 in the laboratory 100, recalculating the processing steps for the test sample 200 by the control unit 110 to determine if and what steps or results were missed; and
performing the recalculated processing steps on the test sample 200.

6. The computer-implemented method according to any of the proceeding claims, further comprising,
if the test sample 200 is located within the laboratory and within the acceptable time period of processing in a laboratory device 400 not connected to a transportation system 340 in the laboratory 100, alerting laboratory operator by the control unit 110 to manually move the test sample 200 to next processing step within the laboratory 100.

7. The computer-implemented method according to any of the proceeding claims, further comprising,
if the test sample 200 is located within the laboratory and within the acceptable time period of processing but a significant number of required tests cannot be performed on the test sample 200 within the laboratory and within acceptable time period of processing, alerting the laboratory operator by the control unit 110 that troubleshooting is needed for that test sample 200 and/or that some laboratory devices 300, 310, 320 need to be masked in order to perform all of the required tests within the acceptable time period.

8. The computer-implemented method according to any of the proceeding claims, further comprising,
if the test sample 200 is located within the laboratory and within the acceptable time period for processing but a significant number of required tests are not available in the laboratory 100, alerting the laboratory operator by the control unit 110 that troubleshooting is needed for that test sample 200 or that the test sample 200 needs to be transported to another laboratory.

9. The computer-implemented method according to claim 8, wherein the non-availability of the required tests comprises lack of needed reagent for performing the required tests on at least one of the laboratory devices 300, 310, 320, lack of adequate calibration for at least one of the laboratory devices 300, 310, 320, lack of available quality control for at least one of the laboratory devices 300, 310, 320, or combinations thereof.

10. The computer-implemented method according to claim 1, wherein if the test sample 200 is located within the laboratory and within the acceptable time period for processing, the historical data of the database 120 is updated with results from the location of the located test sample 200.

11. A computer-implemented method of automatically processing patient test samples in a laboratory setting, the method comprising:
determining by a control unit 110 that a test sample 200 located in a post-analytic device 320 with pending test results will expire before a defined period of time for processing; and
retrieving the test sample 320 from the post-analytic device 320 in order to perform the pending tests before the expiration of the defined period of time for processing.

12. The computer-implemented method according to claim 11, wherein the post-analytic device 320 is an such archive such as a refrigerator.

13. The computer-implemented method according to claims 11 and 12, wherein the test sample 200 is automatically retrieved from the post-analytic device 320 by the laboratory transportation system 340.

14. The computer-implemented method according to claims 11 and 12, wherein the test sample 200 is manually retrieved from the post-analytic device 320 by the laboratory operator after the control unit 110 notifies the laboratory operator of the location of the test sample 200 within the laboratory 100.

15. The computer-implemented method according to claim 10, further comprising:
converting the test sample 200 into a STAT test sample after retrieval from the post-analytic device 320 in order to accelerate the processing of the test sample 200 before the expiration of the defined period of time.
